# EUROPEAN PATENT APPLICATION

(11) **EP 1 832 286 A1**
(43) Date of publication of application: **12.09.2007**
(21) Application number: 05814380.1
(22) Date of filing: 12.12.2005
(51) Int. Cl.: A61K 31/343, A61K 31/519, A61K 45/00, A61P 25/20

(54) **PREVENTIVE OR THERAPEUTIC AGENT FOR SLEEP DISORDER**

(30) Priority: 13.12.2004 JP 2004359487
(71) Applicant: Takeda Pharmaceutical Company Limited, Osaka-shi, Osaka 5408645 (JP)
(72) Inventor: HIRAI, Keisuke TAKEDA PHARMACEUTICAL COMP. Ltd.,, Osaka-shi, Osaka 532-8686 (JP); MIYAMOTO, Masaomi TAKEDA PHARMACEUTICAL COMP.Ltd.,, Osaka-shi, Osaka (JP)
(74) Representative: Rickard, Timothy Mark Adrian
(86) International application number: PCT/JP2005/022756
(87) International publication number: WO 2006/064754

(57) **Abstract**

A combination of (S)-N-[2-(1,6,7,8-tetrahydro-2H-indeno[5,4-b]furan-8-yl)ethyl]propionamide with a medicine capable of prolonging a slow-wave sleep time provides a preventive or therapeutic agent for sleep disorder that induces natural sleep, shortens sleep latency, increases deep sleep, excels in sleep maintenance and ensures an appropriate sleep time.

## Description

### Technical Field

The present invention relates to a preventive or therapeutic agent for sleep disorder.

### Background Art

Almost all of the hypnotics which are used as a therapeutic drug for sleep disorder are benzodiazepine compounds or analogs thereof. However, according to the analysis of electroencephalogram, the sleep induced by benzodiazepine hypnotics is different from natural sleep.

Although ritanserin which is a 5-HT_{2A/2C} receptor antagonist is an effective drug for a treatment of diseases associated with anxiety and depression, it is recently brought to attention as a drug for improving quality of sleep by increasing deep sleep. The sleep induced by ritanserin is more natural than that induced by benzodiazepine hypnotics.

Thus, as a drug for improving quality of sleep by increasing deep sleep, attention is focused on 5-HT_{2A} receptor antagonist, 5-HT_{2C} receptor antagonist, 5-HT_{2A/2C} receptor antagonist, serotonin uptake inhibitor, GABA modulator and GABA uptake inhibitor, and the like.

On the other hand, (S)-N-[2-(1,6,7,8-tetrahydro-2H-indeno[5,4-b]furan-8-yl)ethyl]propionamide disclosed in Patent Publication 1 (hereinafter, sometimes referred to as compound A) has an excellent melatonin agonistic action, therefore this compound is expected as an extremely advantageous preventive or therapeutic agent for sleep disorder which induces more natural sleep than the above-mentioned ritanserin.

The preventive or therapeutic agent for sleep disorder is desired to have properties of, for example, inducing natural sleep, having short sleep latency, increasing deep sleep (i.e., prolonging slow-wave sleep duration), maintaining sleep and ensuring appropriate sleep duration.
[Patent Publication 1] WO 97/32871

### Disclosure of Invention

### Problems to be Solved by the Invention

A drug fully having the above-mentioned desirable properties for a preventive or therapeutic agent for sleep disorder has not been known yet. For example, from the test results of the present inventors, it has been found out that ritanserin increases deep sleep, but shortens total sleep duration.

Thus, the object of the present invention is to provide a preventive or therapeutic agent for sleep disorder that induces natural sleep, shortens sleep latency, increases deep sleep, excels in sleep maintenance, and ensures an appropriate sleep duration.

### Means of Solving the Problems

As a result of intensive studies, the present inventors found out that sleep latency is shortened, deep sleep is increased, excellent maintenance of sleep is obtained, and appropriate sleep duration can be ensured by using a drug for prolonging slow-wave sleep duration in combination with compound A (as a combination of drugs, compounding agent or concomitant drug, or the like), and as a result of further studies, the present invention has been completed.

That is, the present invention provides:
[1] A preventive or therapeutic agent for sleep disorder which comprises a combination of a drug for prolonging slow-wave sleep duration and (S)-N-[2-(1,6,7,8-tetrahydro-2H-indeno[5,4-b]furan-8-yl)ethyl]propionamide;
[2] The preventive or therapeutic agent for sleep disorder according to the above-mentioned [1], wherein the drug for prolonging slow-wave sleep duration is at least one selected from 5-HT_{2A} receptor antagonist, 5-HT_{2C} receptor antagonist, 5-HT_{2A/2C} receptor antagonist, serotonin uptake inhibitor, GABA modulator and GABA uptake inhibitor;
[3] The preventive or therapeutic agent for sleep disorder according to the above-mentioned [1], wherein the drug for prolonging slow-wave sleep duration is at least one selected from M-100907, ritanserin, trazodone, gabapentin, tiagabine, Org-50081 and eplivanserin;
[4] Use of a drug for prolonging slow-wave sleep duration in combination with (S)-N-[2-(1,6,7,8-tetrahydro-2H-indeno[5,4-b]furan-8-yl)ethyl]propionamide for the manufacture of a preventive or therapeutic agent for sleep disorder; and
[5] A method for preventing and/or treating sleep disorder, comprising administering to a mammal in need thereof a therapeutically effective amount of a combination of drug for prolonging slow-wave sleep duration and (S)-N-[2-(1,6,7,8-tetrahydro-2H-indeno[5,4-b]furan-8-yl)ethyl]propionamide; and so on.

### Brief Description of Drawings

Fig. 1 is the graph showing the action of the preventive or therapeutic agent for sleep disorder of the present invention on sleep latency.
Fig. 2 is the graph showing the action of ritanserin on sleep duration.
Fig. 3 is the graph showing the action of compound A on sleep duration.
Fig. 4 is the graph showing the action of the preventive or therapeutic agent for sleep disorder of the present invention on sleep duration.
Fig. 5 is the graph showing the change in wakefulness stage after drug administration. The vertical axis represents the duration (minute) of wakefulness stage, and the horizontal axis represents the elapsed time (hour).
Fig. 6 is the graph showing the change in slow-wave sleep stage after drug administration. The vertical axis represents the duration (minute) of slow-wave sleep stage, and the horizontal axis represents the elapsed time (hour).
Fig. 7 is the graph showing the change in total sleep duration after drug administration. The vertical axis represents the total sleep amount (minute) in 8 hours during which measurement was carried out.

### Best Mode for Carrying Out the Invention

Examples of the drug for prolonging slow-wave sleep duration to be used in the present invention include 5-HT_{2A} receptor antagonist, 5-HT_{2C} receptor antagonist, 5-HT_{2A/2C} receptor antagonist, serotonin uptake inhibitor, GABA modulator and GABA uptake inhibitor.

As the 5-HT_{2A} receptor antagonist, for example, M-100907, Org-50081 and eplivanserin are preferred. M-100907 can be produced by a method described in, for example, Medicinal Chemistry Research, 1996, 6 (pp. 1-10), or an analogous method thereto. Org-50081 can be produced by a method described in, for example, EP 0373998, or an analogous method thereto. In addition, eplivanserin can be produced by a method described in, for example, WO 2005005410, or an analogous method thereto.

As the 5-HT_{2A/2C} receptor antagonist, for example, ritanserin is preferred. Ritanserin can be produced by a method described in, for example, J. Drugs Fut 1986, 11 (5), p. 391, or an analogous method thereto.

As the serotonin uptake inhibitor, for example, trazodone (trazodone hydrochloride) is preferred. Trazodone is commercially available (Bristol-Myers Squibb).

As the GABA modulator, for example, gabapentin is preferred. Gabapentin is commercially available (Pfizer).

As the GABA uptake inhibitor, for example, tiagabine is preferred. Tiagabine is commercially available (Cephalon).

These drugs for prolonging slow-wave sleep duration may be used alone, or as a combination of 2 or more.

Compound A to be used in the present invention can be produced by a method described in, for example, Example 11 of WO 97/32871, or an analogous method thereto.

In the present specification, compounds or drugs may be a free form or a salt, or any one of non-hydrate or hydrate, or may be any one of a racemate or an optically active compound, unless otherwise stated. Such salt is not particularly limited as long as it is a pharmacologically acceptable salt, and examples thereof include a salt with inorganic base, a salt with organic base, a salt with inorganic acid, a salt with organic acid, a salt with basic or acidic amino acid, and the like. Preferable examples of a salt with inorganic base include alkali metal salts such as sodium salt and potassium salt, alkaline earth metal salts such as calcium salt and magnesium salt, aluminum salt, ammonium salt, and the like.

Preferable examples of a salt with organic base include a salt with trimethylamine, triethylamine, pyridine, picoline, 2,6-lutidine, ethanolamine, diethanolamine, triethanolamine, cyclohexylamine, dicyclohexylamine and N,N'-dibenzylethylenediamine, and the like. Preferable examples of a salt with inorganic acid include a salt with hydrochloric acid, hydrobromic acid, nitric acid, sulfuric acid, phosphoric acid, and the like. Preferable examples of a salt with organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, and the like. Preferable examples of a salt with basic amino acid include a salt with arginine, lysine, ornithine, and the like. Preferable examples of a salt with acidic amino acid include a salt with aspartic acid, glutamic acid, and the like.

In the preventive or therapeutic agent for sleep disorder of the present invention, all of the active ingredients may be contained in one preparation, or each or a part of the active ingredients may be compounded in separate preparations. That is, the preventive or therapeutic agent for sleep disorder of the present invention may be, for example, (1) a single preparation wherein active ingredients, i.e., a drug for prolonging slow-wave sleep duration and compound A are formulated, if desired, with an appropriate pharmaceutically acceptable carrier etc., according to a known production method for formulating pharmaceutical preparations, (2) preparations wherein each of the active ingredients, i.e., a drug for prolonging slow-wave sleep duration and compound A is formulated, if desired, with an appropriate pharmaceutically acceptable carrier etc. so as to be used in combination (combined use) simultaneously or at different times, or (3) a combined set (kit products etc.) of preparations wherein each of the active ingredients is separately formulated appropriately with an excipient by a conventional method.

Dosage forms of the preventive or therapeutic agent for sleep disorder of the present invention are not particularly limited, and preferred is a dosage form which can be administered orally to a patient (for example, tablets, fine granules, capsules, granules, etc.). Among them, tablets, fine granules and capsules are particularly preferred.

These preparations of the present invention can be produced by a per se known method (for example, method described in Japanese Pharmacopoeia, etc.) or an analogous method thereto, and a conventionally used pharmacologically acceptable carrier is appropriately used in appropriate amount.

The preventive or therapeutic agent for sleep disorder of the present invention can be used effectively for preventing and/or treating sleep disorder (insomnia) of a mammal (e.g., human, cat, dog, monkey, etc.).

Examples of such sleep disorder include:
(1) intrinsic sleep disorders (e.g., psychophysiological insomnia), extrinsic sleep disorders, and circadian rhythm disorders (e.g., time zone change syndrome (jet lag), shift-work sleep disorder, irregular sleep wake pattern, delayed sleep-phase syndrome, advanced sleep-phase syndrome, non 24-hour sleep-wake disorder);
(2) parasomnias;
(3) sleep disorders associated with medical/psychiatric disorders (e.g., chronic occlusive pulmonary disease, Alzheimer's disease, Parkinson's disease, multiinfarct dementia, schizophrenia, depression, anxiety disorders).

In addition, in the present specification, the sleep disorder (insomnia) includes difficulty falling asleep (sleep-onset insomnia), arousals and awakenings during sleep (sleep maintenance insomnia), early-morning awakening, and a combination thereof.

Further, compound A can be effectively used alone, that is, without combining with a drug for prolonging slow-wave sleep duration for preventing and/or treating sleep disorder (insomnia) of a mammal (e.g., human, cat, dog, monkey, etc.).

Examples of such sleep disorder include:
(1) intrinsic sleep disorders (e.g., psychophysiological insomnia), extrinsic sleep disorders, and circadian rhythm disorders (e.g., time zone change syndrome (jet lag), shift-work sleep disorder, irregular sleep wake pattern, delayed sleep-phase syndrome, advanced sleep-phase syndrome, non 24-hour sleep-wake disorder);
(2) parasomnias;
(3) sleep disorders associated with medical/psychiatric disorders (e.g., chronic occlusive pulmonary disease, Alzheimer's disease, Parkinson's disease, multiinfarct dementia, schizophrenia, depression, anxiety disorders).

The preventive or therapeutic agent for sleep disorder of the present invention is low toxic, and can be safely administered orally to a mammal such as human. The preventive or therapeutic agent for sleep disorder of the present invention can be administered before bedtime, at the moment of waking during the night, and/or at the moment of waking early in the morning depending on the kind of the above-mentioned sleep disorder (insomnia). Furthermore in the same way, when compound A is used alone, the pharmaceutical composition containing compound A can be administered before bedtime, at the moment of waking during the night, and/or at the moment of waking early in the morning depending on the kind of the above-mentioned sleep disorder (insomnia).

The dosage of the preventive or therapeutic agent for sleep disorder of the present invention is varied depending on the subject to be administered, route of administration, disease, kind of active ingredients to be used, and the like. For example, the following amount in terms of dosage of each of active ingredients may be administered per day in a single dose or in divided doses to an adult (body weight about 60kg) of sleep disorder, and it is preferred that each ingredient is administered in combination simultaneously or at different times of from 30 minutes to 3 hours.

When the preventive or therapeutic agent for sleep disorder of the present invention comprising a combination of drug for prolonging slow-wave sleep duration and compound A is used, the dosage for them may be reduced compared to the case when the drug for prolonging slow-wave sleep duration and compound A are used alone, respectively.

For example, in the case of an agent comprising a combination of one kind of drugs for prolonging slow-wave sleep duration and compound A, the single dose of compound A is usually about 0.05 mg to about 10 mg, preferably about 4 mg to about 16 mg, more preferably about 6 mg to about 10 mg.

In addition, the single dose of the drug for prolonging slow-wave sleep duration can be selected in an appropriate amount depending on the kind of the drug, and is usually about 1 mg to about 3,000 mg, and preferably about 5 mg to about 900 mg.

As for more specific compounds, the single dose of M-100907 is usually about 1 mg to about 100 mg, and preferably about 5 mg to about 20 mg.

The single dose of ritanserin is usually about 1 mg to about 100 mg, and preferably about 5 mg to about 20 mg.

The single dose of trazodone is usually about 10 mg to about 500 mg, and preferably about 50 mg to about 300 mg.

The single dose of gabapentin is usually about 50 mg to about 2,400 mg, preferably about 100 mg to about 1,600 mg, and more preferably about 300 mg to about 900 mg.

The single dose of tiagabine is usually about 1 mg to about 100 mg, and preferably about 16 mg to about 56 mg.

Combination ratio (administration ratio) of the drug for prolonging slow-wave sleep duration and compound A in the preventive or therapeutic agent for sleep disorder of the present invention is usually about 0.1 to about 400 : 1, and preferably about 0.1 to about 30 : 1 (weight ratio). In particular, when the drug for prolonging slow-wave sleep duration is gabapentin, the combination ratio (administration ratio) of gabapentin and compound A is usually about 0.1 to about 400 : 1, preferably about 8 to about 400 : 1, more preferably about 20 to about 200 : 1, and most preferably about 80 to about 120 : 1.

Furthermore, the preventive or therapeutic agent for sleep disorder of the present invention can be jointly used in combination with other active ingredients as long as its advantageous property is substantially not interfered. The other active ingredients, drug for prolonging slow-wave sleep duration and compound A may be blended according to a per se known method to give a pharmaceutical composition (e.g., tablets, powders, granules, capsules (including soft capsules), liquids, injections, suppositories, sustained-release preparations, etc.), and the obtained composition may be administered, or preparations formulated separately may be administered to the same subject simultaneously or at different times in the same way of the preparation of the present invention.

The present invention will be described in detail through the following Experiments and Preparation Examples. However, these are just an example, and the present invention is not limited by the examples, and may be changed without departing from the scope of the present invention. In the following examples, compound A was prepared to use by a method described in WO 97/32871. In addition, ritanserin was obtained from Sigma-Aldrich Co.

### Experiment 1

### [Method]

3 Female Macaca fascicularis were used for experiment. Drugs were administered according to crossover design. Test animals were operated to place an electrode to measure electroencephalogram, and were naturalized fully to test cage. The test animals were raised under light-dark cycle of 12 hour-light period and 12 hour-dark period (light period is from 6 : 00 to 18 : 00).

The dose of compound A was 0.3 mg/kg, and the dose of ritanserin was 1 mg/kg, and these were suspended in 0.5% methylcellulose solution to make dosage of 1 ml/kg. As control, 0.5% methylcellulose solution was used. Administration was conducted orally using transnasal catheter 5 to 10 minutes before extinction (17 : 50 - 17 : 55).

Measurement items were electroencephalogram, ocular movement and myogenic potential, and behavior was observed using infrared camera. Measurement was carried out for 12 hours from lights-out (18 : 00) to lights-on (6 : 00).

Regarding the condition of sleep and wakefulness of the animals, sleep stage was classified into wakefulness, light NREM (stage 1 and 2: light sleep, LS), slow-wave sleep (stage 3 and 4: SWS) and REM sleep based on these results according to international classification of human sleep electroencephalogram of Rechtschaffen and Kales (1968).

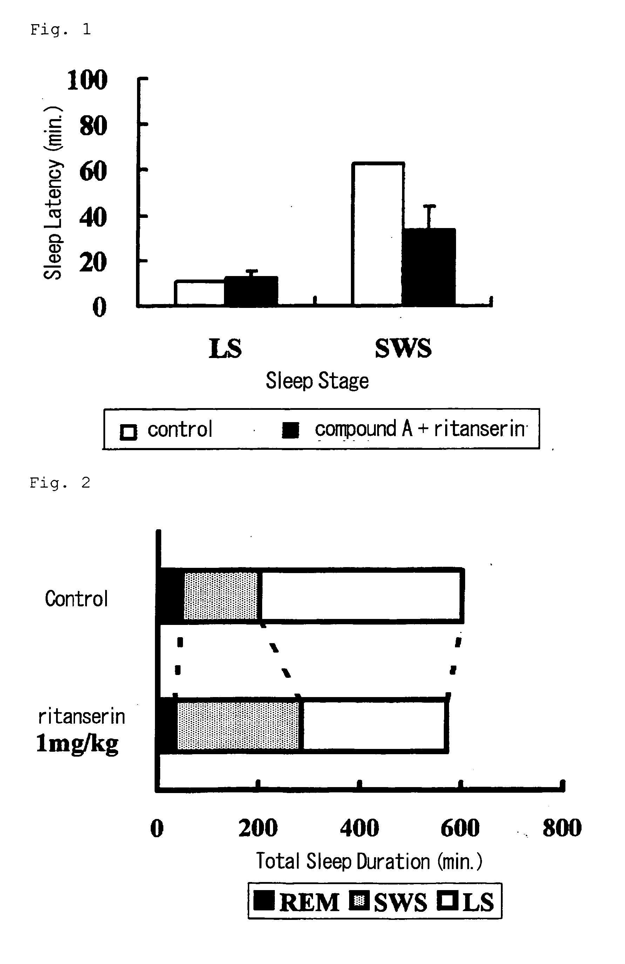

Data analysis was carried out for sleep latency and total sleep duration in the night. The results were shown in Figures 1 to 4.

### [Result]

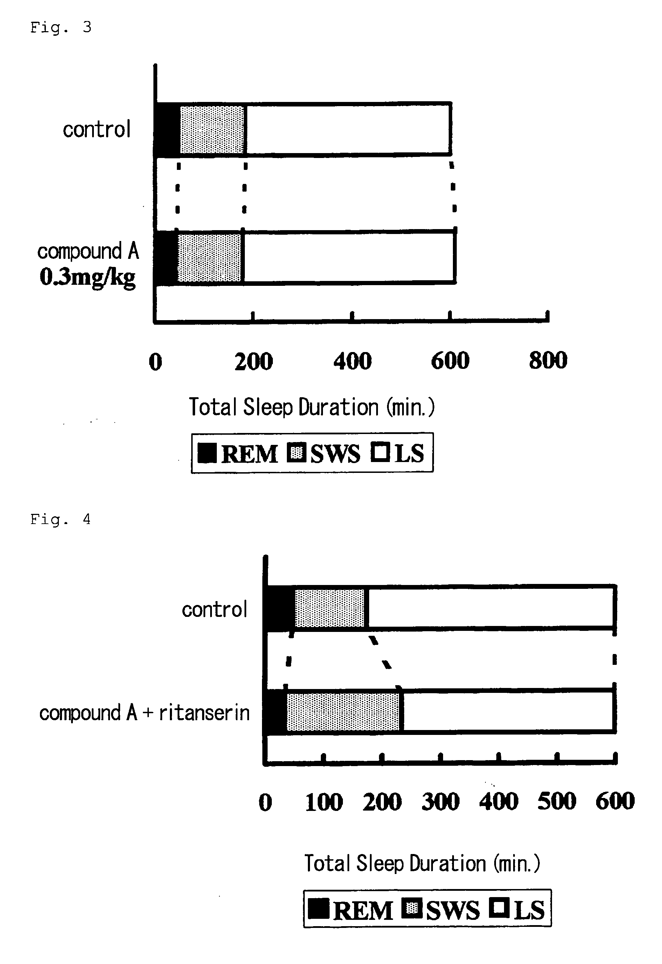

In sole administration of ritanserin, sleep latency of LS was reduced from 18.7 minutes to 12.0 minutes, and sleep latency of SWS was reduced from 67.3 minutes to 39.3 minutes. In addition, in breakdown of sleep duration, SWS was increased from 152 minutes to 249 minutes, and an action of increasing deep sleep was confirmed, but there was seen a tendency to reduce total sleep duration from 602 minutes to 573 minutes (Figure 2). In sole administration of compound A, sleep latency of LS was reduced from 15.0 minutes to 13.3 minutes, and sleep latency of SWS was reduced from 57.7 minutes to 31.3 minutes. In addition, in breakdown of sleep duration, SWS was almost unchanged, i.e., reduced from 136 minutes to 135 minutes, and there was seen a tendency to slightly increase total sleep duration from 601 minutes to 610 minutes (Figure 3). When these two drugs were used jointly, sleep latency of LS was slightly increased from 10.3 minutes to 12.7 minutes. This case is supposed that the vehicle control group happened to have short sleep latency, and the sleep latency of the drug-treated group remained almost the same as that of vehicle control groups tested before. At the same time, sleep latency of SWS was reduced from 62.7 minutes to 33.3 minutes (Figure 1). In addition, in breakdown of sleep duration, SWS was increased from 127 minutes to 200 minutes, an action of increasing deep sleep was confirmed, and total sleep duration was maintained from 599 minutes to 599 minutes (Figure 4). From the above results, it was found out that although ritanserin having an action of increasing deep sleep prolongs SWS sleep duration, there is seen a tendency to reduce total sleep duration, and the sole administration of compound A shows an action of hastening initiation of sleep without changing sleep phase, and by using both together, an ideal profile of sleep can be obtained in which while accelerating initiation of sleep, deep sleep phase is increased and further total sleep duration is maintained.

### Experiment 2

### Effect of combined use of compound A and gabapentin (GBP) on sleep wakefulness of cat

### [Method]

### 1. Implantation of a chronic electrode

Used animals: 4 Siamese cats (purchased from Narc co.), 2 European shorthairs (purchased from Nisseiken co. Ltd.) and 1 mongrel cat (purchased from Narc co.) were used.

Under pentobarbital anesthesia, the following electrode and the like were implanted. An antibiotic was administered to prevent bacterial infection, and taming to cage for measuring electroencephalogram was started from 3 to 4 days after the operation. Measurement of electroencephalogram was carried out after 1 to 2 weeks of taming.
- electrode for recording electroencephalogram in frontal lobe of cerebral cortex, frontal lobe and hippocampus
- electrode for electrooculogram in orbit bone
- stainless wire for recording electromyogram in dorsal cervical muscle

### 2. Drug administration group

1) Compound A (0.1 mg/kg) + Vehicle (0.5% methylcellulose in distilled water)
2) Compound A (0.1 mg/kg) + Gabapentin (10 mg/kg)
   Vehicle and drugs to be administered were filled in a capsule (capsule No. 1 of Japanese Pharmacopeia), and it was compulsorily administered orally. Treated groups were comprised of 7 animals per one group, and crossover test was carried out.

### 3. Schedule for administration and measurement of sleep electroencephalogram

After being attached the electrodes for measurement from about 8 : 30 in the morning, the cats were placed in test box. Administration was conducted by 9 : 55 to 10 : 05 in the morning, and the sleep electroencephalogram for 8 hours after administration was measured.

### 4. Electroencephalogram measurement and electroencephalogram analysis

Electroencephalogram data was obtained using Synafit 2500 of NEC Saneisha. Sleep Sign Ver. 2.0 that is a program for sleep analysis study of Kissei Comtec Co. LTD., was used for analysis. As characteristic parameter, δ wave was set by the waveform derived from cortex and θ wave was set by the waveform derived from hippocampus. Analysis was carried out every 20 seconds with If ... Then method using this parameter, and Wakefulness, Slow-Wave Sleep (SWS) and REM Sleep (REM) were automatically judged. Furthermore, after completion of these automatic judgments, visual judgment was conducted and the judgment was modified when the judgment of sleep stage was apparently different. In addition, the stage judged to be at slow-wave sleep was extracted, frequency analysis was carried out, and power spectrum during slow-wave sleep was calculated.
a≤ EMG Integral ≤100 → Yes Wakefulness
↓ No
20≤ Delta % Time ≤100 & 0≤ EMG Integral <b → Yes SWS
↓ No
25≤ Theta % Time ≤100 & 0≤ EMG Integral <c → Yes REM
↓ No
3≤ fast wave% Time ≤100 → Yes Wakefulness
↓ No
Previous Stage (reflect immediately preceding judgment of sleep)
* values of a, b, c should be determined with an appropriate numerical value for each body.

### 5. Statistical Analysis

Method of a two-period, two-treatment crossover test of SAS preclinical package was applied to statistical analysis, and the case when hazard ratio was *P<0.05 was judged to be statistically significant.

### [Result]

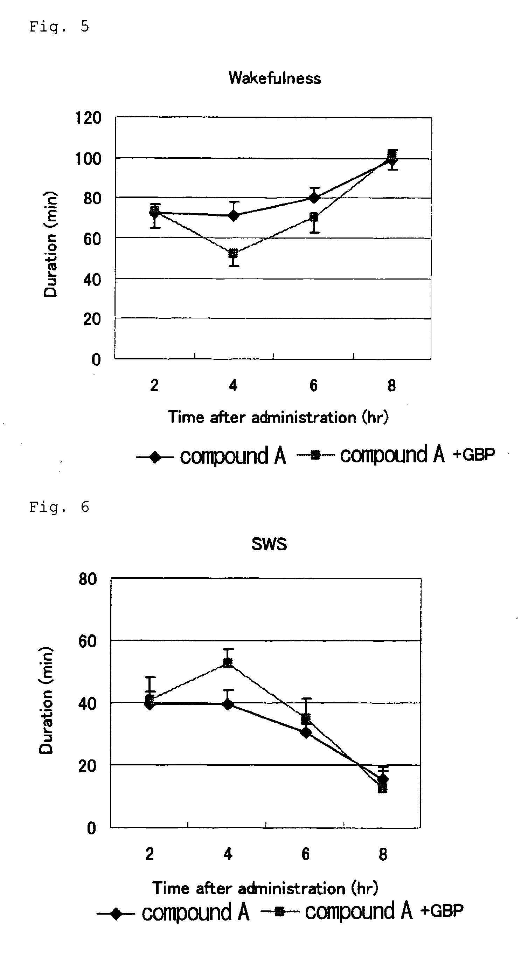

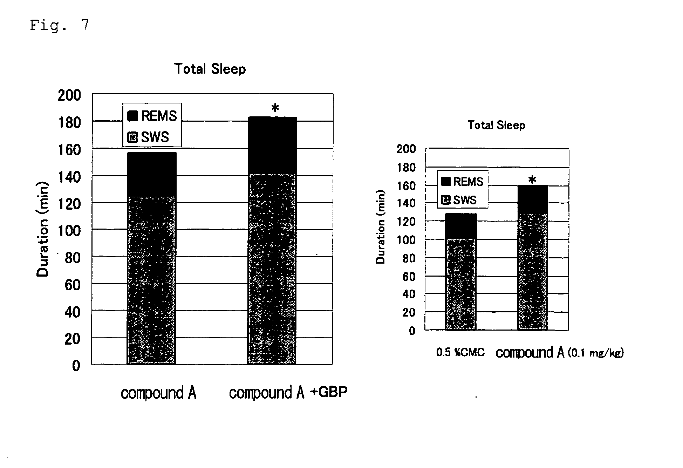

Changes of each sleep stage after drug administration were shown in Figures 5 to 7. In the sole administration group of compound A, an increase in slow-wave sleep duration of about 4 hours from 2 hours after administration was observed compared to the group of combined use with GBP (total slow-wave sleep duration: 124.9 minutes → 142.1 minutes). Furthermore, in the group of combined use with GBP, total sleep duration was increased significantly compared to the sole administration group of compound A (Figure 7). From the above results, it was found out that the effects of the combined use of compound A and GBP were observed to be much higher in terms of increase in slow-wave sleep duration and increase in total sleep duration than the effects of sole compound A, and good profile of sleep can be obtained. In addition, from the knowledge about time zone in which an effect on increase in slow-wave sleep duration is seen, an inhibitory effect on arousal during sleep is observed.

### Preparation Example 1

| | |
|---|---|
| (1) Ritanserin | 10.0 g |
| (2) Compound A | 10.0 g |
| (3) Lactose | 60.0 g |
| (4) Corn Starch | 35.0 g |
| (5) Gelatin | 3.0 g |
| (6) Magnesium stearate | 2.0 g |

A mixture of ritanserin 10.0 g, compound A 10.0 g, lactose 60.0 g and corn starch 35.0 g was granulated through 1 mm mesh sieve using 10% by weight aqueous solution of gelatin 30 ml (3.0 g as gelatin), and the granules were dried at 40°C, and passed through the sieve again. The resulting granules are mixed with magnesium stearate 2.0 g, and the mixture is compressed. The resulting core tablets are sugar-coated using a suspension of sucrose, titanium oxide, talc and gum arabic in water. The coated tablets are burnished with yellow beeswax to give 1,000 coated tablets.

### Industrial Applicability

According to the present invention, there is provided a preventive or therapeutic agent for sleep disorder that induces natural sleep, shortens sleep latency, increases deep sleep, excels in sleep maintenance, and ensures an appropriate sleep duration.

## Claims

1. A preventive or therapeutic agent for sleep disorder which comprises a combination of a drug for prolonging slow-wave sleep duration and (S)-N-[2-(1,6,7,8-tetrahydro-2H-indeno[5,4-b]furan-8-yl)ethyl]propionamide.

2. The preventive or therapeutic agent for sleep disorder according to claim 1, wherein the drug for prolonging slow-wave sleep duration is at least one selected from 5-HT_{2A} receptor antagonist, 5-HT_{2C} receptor antagonist, 5-HT_{2A/2C} receptor antagonist, serotonin uptake inhibitor, GABA modulator and GABA uptake inhibitor.

3. The preventive or therapeutic agent for sleep disorder according to claim 1, wherein the drug for prolonging slow-wave sleep duration is at least one selected from M-100907, ritanserin, trazodone, gabapentin, tiagabine, Org-50081 and eplivanserin.

4. Use of a drug for prolonging slow-wave sleep duration in combination with (S)-N-[2-(1,6,7,8-tetrahydro-2H-indeno[5,4-b]furan-8-yl)ethyl]propionamide for the manufacture of a preventive or therapeutic agent for sleep disorder.

5. A method for preventing and/or treating sleep disorder, comprising administering to a mammal in need thereof a therapeutically effective amount of a combination of drug for prolonging slow-wave sleep duration and (S)-N-[2-(1,6,7,8-tetrahydro-2H-indeno[5,4-b]furan-8-yl)ethyl]propionamide.
